# EUROPÄISCHE PATENTSCHRIFT

(11) **EP 1 401 802 B1**
(45) Veröffentlichungstag und Bekanntmachung des Hinweises auf die Patenterteilung: **30.04.2008**
(21) Anmeldenummer: 02745367.9
(22) Anmeldetag: 06.06.2002
(51) Int. Cl.: C07C 263/10

(54) **VERFAHREN ZUR HERSTELLUNG VON ISOCYANATEN**
METHOD FOR PRODUCING ISOCYANATES
PROCEDE DE PRODUCTION D'ISOCYANATES

(30) Priorität: 19.06.2001 DE 10129233
(43) Veröffentlichungstag der Anmeldung: 31.03.2004
(73) Patentinhaber: BASF SE, 67056 Ludwigshafen (DE)
(72) Erfinder: WÖLFERT, Andreas, 74906 Bad Rappenau (DE); MÜLLER, Christian, 68167 Mannheim (DE); STRÖFER, Eckhard, 68163 Mannheim (DE)
(86) Internationale Anmeldenummer: PCT/EP2002/006196
(87) Internationale Veröffentlichungsnummer: WO 2002/102763

(56) Entgegenhaltungen:
- WO-A-96/16028
- DE-B- 1 192 641

## Beschreibung

Die vorliegende Erfindung betrifft ein Verfahren zur Herstellung von Isocyanaten durch Umsetzung von Aminen mit Phosgen unter Verwendung von Isocyanat als Lösungsmittel, wobei das als Lösungsmittel verwendeten Isocyanat der Umsetzung erst nach dem Zusammenführen von Amin und Phosgen zugegeben wird.

Es sind bereits verschiedene Verfahren zur Herstellung von Isocyanaten durch Umsetzung von Aminen mit Phosgen in Gegenwart von Isocyanat bekannt.

DE-A-1192641 beschreibt ein Verfahren zur Herstellung von Isocyanaten durch Umsetzung von Aminen mit Phosgen, wobei das Isocyanat, welches in der Umsetzung hergestellt wird, als Lösungsmittel für Phosgen verwendet wird.

DE-A-2252068 beschreibt ein Verfahren zur Herstellung von organischen Isocyanaten, dadurch gekennzeichnet, dass Amin mit vorerhitztem Phosgen in Gegenwart eines Überschusses an Isocyanat bei solchen Temperaturen und Drücken zur Reaktion gebracht wird, dass eine homogene flüssige Phase vorliegt.

US-A-2 822 373 beschreibt einen kontinuierlichen Prozess zur Herstellung von. Isocyanaten, bei dem eine Phosgenlösung mit einer Lösung eines organischen Amins in einem turbulent durchströmten Reaktorkreislaufsystem gemischt wird. Die frische Phosgenlösung wird hierbei vor der Vermischung mit der Aminlösung mit der im Kreislauf geführten Reaktionslösung vereinigt.

WO 96/16028 beschreibt ein kontinuierliches Verfahren zur Herstellung von Isocyanaten durch Umsetzung von entsprechenden primären Aminen mit Phosgen in Gegenwart eines Isocyanats als Lösungsmittel, wobei das Amin mit Phosgen, das im Isocyanat zu 10 bis 60 Gew.% gelöst ist, umgesetzt wird.

Es ist weiterhin bekannt, dass die Verwendung eines hohen Phosgenüberschusses gegenüber den eingesetzten Aminogruppen zu hohen Selektivitäten bezüglich des hergestellten Isocyanates führt und somit einen entscheidenden Einfluss auf die Wirtschaftlichkeit des Verfahrens haben kann. Mit zunehmendem Verhältnis von Phosgen zu Aminogruppen steigt jedoch der Phosgen-Hold-Up der Anlage, wobei aufgrund der Giftigkeit von Phosgen ein möglichst geringer Phosgen-Hold-Up der Anlage anges-trebt wird.

Aufgabe der Erfindung ist es daher, ein Verfahren zur Herstellung von Isocyanaten bereitzustellen, welches es gestattet, die Umsetzung unter hoher Selektivität durchzuführen, ohne dass jedoch der Phosgen-Hold-Up erhöht oder die Raum-Zeit-Ausbeute erniedrigt wird.

Die Aufgabe der Erfindung wurde unerwartet dadurch gelöst, dass bei der Herstellung von Isocyanaten durch Umsetzung von Amin und Phosgen ein Isocyanat als Lösungsmittel zugesetzt wird, wobei dieses Lösungsmittel nicht, wie im Stand der Technik beschrieben, vor der Reaktion dem Phosgen zugesetzt wird, sondern erst nach dem Zusammenbringen von Phosgen und Amin der Reaktion zugeführt wird.

Gegenstand der Erfindung ist daher ein Verfahren zur Herstellung von Isocyanaten durch Umsetzung von primären Aminen mit Phosgen, wobei Isocyanat als Lösungsmittel verwendet wird, dadurch gekennzeichnet, dass ein Anteil oder die gesamte Menge des als Lösungsmittel verwendeten Isocyanats den Reaktionspartnern der Umsetzung erst nach dem Zusammenführen von Amin und Phosgen zugegeben wird.

Das erfindungsgemäße Verfahren umfasst kontinuierliche, halbkontinuierliche und diskontinuierliche Verfahren. Bevorzugt sind kontinuierliche Verfahren.

Bei der Herstellung von Isocyanat durch Umsetzung von einem primären Amin mit Phosgen bildet sich gemäß nachstehendem Reaktionsschema in einem ersten schnellen Schritt intermediär Carbamylchlorid, das sich im Geschwindigkeit bestimmenden, langsamen Schritt in einer Gleichgewichtsreaktion zu Isocyanat und HCl zersetzt.

R-NH₂ + COCl₂ → R-NH-COCl + HCl ⇔ R -NCO + 2 HCl,

wobei R ein organischer Rest ist.

Ferner kann der entstehende Chlorwasserstoff mit Aminen zu Aminhydrochloriden reagieren.

Für das erfindungsgemäße Verfahren ist es nun wesentlich, dass die Zugabe des Isocyanats als Lösungsmittel erst dann erfolgt, wenn bereits Amin und Phosgen miteinander in Kontakt gebracht worden sind, bevorzugt vermischt worden sind. In einer erfindungsgemäßen Ausführungsform wird das Lösungsmittel zu einem Zeitpunkt bzw. an einer Stelle zugesetzt, wo bereits mindestens 50 % der eingesetzten Aminogruppen, bevorzugt mindestens 80 %, mehr bevorzugt mindestens 90 %, insbesondere mindestens 95 %, umgesetzt wurden.

Die Geschwindigkeit der Umsetzung von Phosgen mit Amin bzw. von Chlorwasserstoff mit Amin hängt vor allem von der Art des herzustellenden Isocyanats und der gewählten Reaktionstemperatur ab. Entsprechend kann die Zugabe des Isocyanats als Lösungsmittel zeitlich zwischen 0,1 Millisekunden und 10 Minuten nach dem Zusammenbringen von Phosgen und Amin erfolgen.

Bei dem erfindungsgemäßen Verfahren wird ein Anteil oder die gesamte Menge des als Lösungsmittel verwendeten Isocyanats der Umsetzung, d.h. den Reaktionspartnern Amin und Phosgen, erst nach dem Zusammenführen von Amin und Phosgen zugegeben. In einer bevorzugten Ausführungsform beträgt der Anteil, welcher der Umsetzung erst nach dem Zusammenführen von Amin und Phosgen zugegeben wird, mindestens 25 Gew.-%, bevorzugt mindestens 50 Gew.-%, stärker bevorzugt mindestens 75 Gew.-%, insbesondere mindestens 90 Gew.-%, bezogen auf die gesamte Menge Isocyanat, die als Lösungsmittel eingesetzt wird. Der Anteil, der nicht erst nach dem Zusammenführen von Amin und Phosgen zugegeben wird, kann bereits vor der Umsetzung dem Amin oder bevorzugt dem Phosgen zugesetzt werden.

Für das erfindungsgemäße Verfahren kann ein beliebiges primäres Amin oder ein Gemisch aus zwei oder mehr dieser Amine eingesetzt werden. Bevorzugt werden aromatische Amine, insbesondere solche der Diaminodiphenylmethanreihe oder deren höheren Homologen eingesetzt. Beispiele sind Methylendiphenylamin (MDA; einzelne Isomere, Isomerengemisch und/oder Oligomere davon), Toluylendiamin (TDA), n-Pentylamin, 6-Methyl-2-heptanamin, Cyclopentylamin, R,S-1-Phenylethylamin, 1-Methyl-3-phenylpropylamin, 2,6-Xylidin, 2-(N,N-Dimethylamino)ethylamin, 2-(N,N-Diisopropylamino)ethylamin, C11-Neodiamin, Isophorondiamin, 3,3'-Diaminodiphenylsulfon und 4-Aminomethyl-1,8-octandiamin. Bevorzugt verwendet werden MDA und TDA.

Das erfindungsgemäße Verfahren eignet sich entsprechend zur Herstellung von beliebigen Isocyanaten. Besonders vorteilhaft kann das Verfahren zur Herstellung von Methylen(diphenyldiisocyanat) (MDI) und Toluylendiisocyanat (TDI) angewandt werden.

Das als Lösungsmittel verwendete Isocyanat ist bevorzugt das herzustellende Isocyanat. Es kann aus einer externen Quelle stammen oder bevorzugt aus dem erfindungsgemäßen Verfahren entnommen und rückgeführt werden. Alternativ können jedoch auch andere geeignete Isocyanate, gegebenenfalls auch im Gemisch, als Lösungsmittel verwendet werden.

Dem erfindungsgemäßen Verfahren kann ein zusätzliches inertes Lösungsmittel beigesetzt werden. Dieses zusätzliche inerte Lösungsmittel ist üblicherweise ein organisches Lösungsmittel oder Gemische davon. Dabei sind Chlorbenzol, Dichlorbenzol, Trichlorbenzol, Toluol, Hexan, Diethylisophthalat (DEIP), Tetrahydrofuran (THF), Dimethylformamid (DMF), Benzol und deren Gemische bevorzugt. Besonders bevorzugt ist Chlorbenzol. Das zusätzliche inerte Lösungsmittel kann bevorzugt zu Beginn der Umsetzung dem Amin zugesetzt werden. Das inerte Lösungsmittel wird üblicherweise in einer Menge von 5 bis 1000 Gew.-%, bevorzugt 50 bis 500 Gew.-%, bezogen auf die Menge an eingesetzten Amin, verwendet.

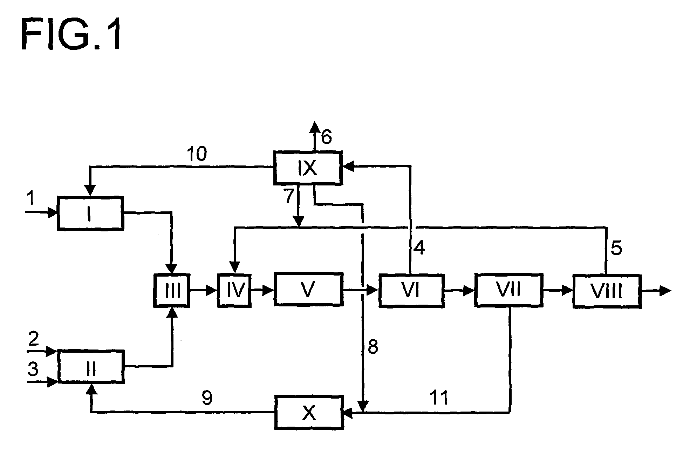

Das erfindungsgemäße Verfahren soll anhand eines allgemeinen Verfahrensschemas für ein kontinuierliches Verfahren gemäß Figur 1 näher erläutert werden. In Figur 1 bedeutet:
- I: Phosgenvorlage
- II: Aminvorlage
- III: Erste Mischvorrichtung
- IV: Zweite Mischvorrichtung
- V: Reaktionsvorrichtung
- VI: Erste Trennvorrichtung
- VII: Zweite Trennvorrichtung
- VIII: Isocyanatvorlage
- IX: Phosgenaufarbeitung
- X: Lösungsmittelaufarbeitung
- 1: Zufuhr Phosgen
- 2: Zufuhr Amin
- 3: Zufuhr inertes Lösungsmittel
- 4: Abgetrennter Chlorwasserstoff und inertes Lösungsmittel
- 5: Rückgeführter Isocyanatstrom
- 6: Ausgetragener Chlorwasserstoff
- 7: Abgetrenntes Isocyanat
- 8, 11: Abgetrenntes inertes Lösungsmittel
- 9: Aufgearbeitetes inertes Lösungsmittel
- 10: Aufgearbeitetes Phosgen

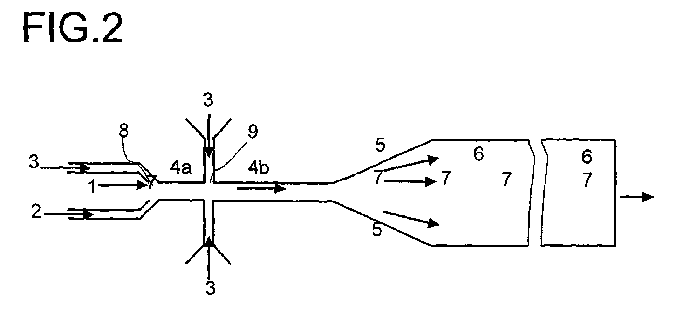

Das Amin aus der Aminvorlage II und Phosgen, aus der Phosgenvorlage I werden in einer geeigneten ersten Mischvorrichtung III vermischt. Das Gemisch aus Amin und Phosgen wird in einer zweiten Mischvorrichtung IV mit Isocyanat als Lösungsmittel vermischt und in die Reaktionsvorrichtung V überführt. Geeignete Mischvorrichtungen sind beispielsweise Düsen oder Reaktionsmischpumpen. Ebenfalls ist es möglich, die beiden Vermischungen in den Mischvorrichtungen III und IV in einer gemeinsamen Mischvorrichtung, jedoch in räumlich getrennten Bereichen durchzuführen, wie nachstehend anhand der bevorzugten Ausführungsform gemäß Figur 2 erläutert wird.

Nach dem Vermischen wird das Gemisch in eine Reaktionsvorrichtung V überführt. Geeignete Reaktionsvorrichtungen sind beispielsweise Rohrreaktoren, Turmreaktoren, Reaktionskessel oder Reaktionskolonnen. Rohrreaktoren sind bevorzugt. Ebenfalls sind Vorrichtungen verwendbar, die sowohl Misch- als auch Reaktionsvorrichtung darstellen, beispielsweise Rohrreaktoren mit angeflanschten Düsen.

Bei den beiden Trennvorrichtungen VI und VII handelt es sich bevorzugt um Destillationseinheiten. In der ersten Trennvorrichtung VI wird üblicherweise vom Isocyanatstrom Chlorwasserstoff und gegebenenfalls inertes Lösungsmittel und/oder geringe Anteile des Isocyanatstroms abgetrennt. In der zweiten Trennvorrichtung VII wird bevorzugt inertes Lösungsmittel abgetrennt und anschließend aufgearbeitet (X) und der Aminvorlage II wieder zugeführt.

Eine bevorzugte Ausführungsform des erfindungsgemäßen Verfahrens umfasst, wie in Figur 2 veranschaulicht, die Verwendung einer nachstehend beschriebenen Mischvorrichtung, an die sich unmittelbar nachfolgend eine Reaktionsvorrichtung anschließt.

Figur 2 veranschaulicht einen bevorzugten Aufbau für das erfindungsgemäße Verfahren. In der Figur 2 bedeutet:
- 1: Aminzufuhr
- 2: Phosgenzufuhr
- 3: Isocyanatzufuhr
- 4a: Mischrohr
- 4b: Mischrohr
- 5: Diffusor
- 6: Rohrreaktor
- 7: Achse
- 8: Ringspalt der Phosgeneinleitung
- 9: Ringspalt der Isocyanateinleitung

Bei der kontinuierlich ablaufenden Phosgenierung wird nun das Amin oder die Aminlösung 1 auf der Achse 7 in die Mischvorrichtung, üblicherweise mit Geschwindigkeiten von 5 bis 60 m/s, eingedüst. Das Phosgen oder die Phosgenlösung gelangen über einen Ringspalt der Phosgeneinleitung 8 mit Geschwindigkeiten, die ebenfalls im Bereich von 5 bis 60 m/s liegen können, in die Mischvorrichtung. Die beiden Ströme von Amin und Phosgen werden vereinigt (entspricht der ersten Mischvorrichtung in Figur 1), über ein optionales Mischrohr 4a geführt und anschließend wird über den Ringspalt der Isocyanateinleitung 9 Isocyanat zugeführt (entspricht der zweiten Mischvorrichtung in Figur 1). Nach einem optionalen Mischrohr 4b wird das Reaktionsgemenge über den Diffusor 5 in den Rohrreaktor 6 geführt. Nach ein Verweilzeit von 10 s bis 20 min wird die entstandene Rohisocyanatlösung dem Rohrreaktor entnommen.

In einer bevorzugten Ausführungsform wird als Mischdüseneinrichtung eine axialsymmetrische Mischrohreinrichtung mit einer axialen Aminzufuhr und einer Phosgen- und Isocyanatzufuhr, die über zwei nicht axial angeordnete Ringspalte erfolgt, verwendet.

Der für das erfindungsgemäße Verfahren vorteilhafte Temperaturbereich hängt unter anderem von der Art und der Menge des Lösemittels und von dem herzustellenden Isocyanat ab. Im allgemeinen liegt in der Mischeinheit eine Temperatur zwischen -20°C und 300°C, bevorzugt zwischen 10°C und 200°C und besonders bevorzugt zwischen 80°C und 150°C vor. Die Temperatur im Reaktor liegt im allgemeinen zwischen 10°C und 360°C und bevorzugt zwischen 40°C und 210°C und besonders bevorzugt bei 100°C und 180°C. Ferner liegt im allgemeinen der Absolutdruck zwischen 0,2 bar und 50 bar, bevorzugt zwischen 1 bar und 25 bar, besonders bevorzugt zwischen 3 und 17 bar vor.

Die Verweilzeit der Flüssigkeit in der Mischvorrichtung und im Reaktor liegt in der Summe zwischen 12 s und 20 min, bevorzugt im Bereich von 36 s bis 16 min, und besonders bevorzugt zwischen 60 s und 12 min.

Das molare Verhältnis von eingesetztem Phosgen zu Aminogruppen beträgt 1:1 bis 12:1, bevorzugt 1,1:1 bis 4:1. Amin und Phosgen können lösungsmittelfrei oder in einem oder mehreren der oben genannten inerten Lösungsmitteln gelöst zur Anwendung kommen. Alternativ kann das Phosgen auch gasförmig in die Aminlösung eingedüst werden. Zusätzlich kann wie vorstehend beschrieben Phosgen auch mit einem Anteil des als Lösungsmittel verwendeten Isocyanats vorgemischt werden.

Die im erfindungsgemäßen Verfahren eingesetzte Menge des als Lösungsmittel verwendeten Isocyanats beträgt im allgemeinen 10 bis 1000 Gew.-%, bevorzugt 50 bis 500 Gew.-%, mehr bevorzugt 100 bis 400 Gew.-%, bezogen auf die eingesetzte Menge an Phosgen.

Nach der Reaktion wird das Stoffgemisch bevorzugt mittels Rektifikation in Isocyanat, Lösungsmittel, Phosgen und Chlorwasserstoff aufgetrennt. Geringe Mengen von Nebenprodukten, die im Isocyanat verbleiben, können mittels zusätzlicher Rektifikation oder auch Kristallisation vom erwünschten Isocyanat getrennt werden.

Je nach Wahl der Reaktionsbedingungen kann das rohe Endprodukt inertes Lösungsmittel, Carbamoylchlorid und/oder Phosgen enthalten und nach den bekannten Methoden weiterverarbeitet werden (siehe z.B. WO 99/40059). Ferner kann es vorteilhaft sein, das Produkt bei Entnahme über einen Wärmetauscher zu leiten.

Die Erfindung soll durch nachfolgende Beispiele veranschaulicht werden.

### Beispiel 1

90 g eines Toluylendiamingemisches (TDA-Gemisch), das zu 80 Gewichts-% aus 2,4-TDA und zu 20 Gewichts-% aus 2,6-TDA bestand, wurde in 360 g Monochlorbenzol (MCB) gelöst. Die erhaltene TDA-Lösung wurde in der in Figur 2 dargestellte Mischvorrichtung auf der Achse mit einer Geschwindigkeit von 30 m/s und einem Durchsatz von 1,8 1/h bei einer Temperatur von 50°C eingedüst. Gleichzeitig wurden 1,6 kg einer 25 %igen Phosgenlösung, die aus 400 g Phosgen und 1,2 kg MCB hergestellt wurde, mit einer Geschwindigkeit von 30 m/s und unter einem Winkel von 45° gegenüber der Achse über den ersten Ringspalt der Düsenmischvorrichtung bei einer Temperatur von 30°C eingedüst. Anschließend wurden 1,2 kg Toluylendiisocyanat, das zu 80 Gewichts-% aus 2,4-TDI und zu 20 Gewichts-% aus 2,6-TDI besteht, über den zweiten Ringspalt unter einem Winkel von 90° gegenüber der Achse der Mischvorrichtung mit einer Geschwindigkeit von 25 m/s in der Durchtrittsfläche in das axiale Mischrohr und einer Temperatur von 30°C eingedüst. Das Gemisch gelangte unmittelbar aus der Düsenmischvorrichtung in einen Rohrreaktor, der ein Volumen von ca. 300 ml und eine Länge von 24 m hatte und für das Gesamtgemisch eine Verweilzeit von ca. 1,4 min besaß und auf eine Temperatur von ca. 130° bis 140° temperiert war. Vor und nach der Synthese wurde die Apparatur kontinuierlich mit Monochlorbenzol als inertem Lösungsmittel vorgespült. Nach der destillativen Entfernung des Phosgens und des Chlorbenzols wurde TDI bei einer Reinheit von ca. 99,2 % (GC) und einer Ausbeute von 99,1% gewonnen. Der Phosgen-Hold-Up im Reaktor betrug auf Basis des eintretenden Phosgen-Massenstromes maximal ca. 38 g. Dieser Wert wurde mit der Annahme errechnet, dass das Phosgen in der Reaktion nicht verbraucht wurde. Damit ergab sich ein Phosgen-Hold-Up von ca. 76 g für eine Produktionsleistung von 1 kg TDI pro Stunde.

### Vergleichsbeispiel 2 unter Verwendung einer Isocyanat-Phosgen-Lösung

Analog zu Beispiel 1 wurden 90 g eines Toluylendiamingemisches (TDA-Gemisch), das zu 80 Gewichts-% aus 2,4-TDA und zu 20 Gewichts-% aus 2,6-TDA bestand, in 360 g Monochlorbenzol gelöst. Die erhaltene TDA-Lösung wurde in der in Figur 2 dargestellte Mischvorrichtung auf der Achse mit einer Geschwindigkeit von 30 m/s und einem Durchsatz von 1,8 1/h bei einer Temperatur von 50°C eingedüst. Gleichzeitig wurden 1,6 kg einer 25 %-Phosgenlösung, die aus 400 g Phosgen und 1,2 kg Toluylendiisocyanat, das zu 80 Gewichts-% aus 2,4-TDI und zu 20 Gewichts-% aus 2,6-TDI besteht, hergestellt wurde, mit einer Geschwindigkeit von 30 m/s und unter einem Winkel von 45° gegenüber der Achse über den ersten Ringspalt der Düsenmischvorrichtung bei einer Temperatur von 30°C eingedüst. Das Gemisch gelangte unmittelbar aus der Düsenmischvorrichtung in einen Rohrreaktor, der ein Volumen von ca. 180 ml und eine Länge von 14,4 m hatte und für das Gesamtgemisch eine Verweilzeit von ca. 1,4 min besaß und auf eine Temperatur von ca. 130° bis 140° temperiert war. Vor und nach der Synthese wurde die Apparatur kontinuierlich mit Monochlorbenzol als inertes Lösungsmittel vorgespült. Nach der destillativen Entfernung des Phosgens und des Chlorbenzols wurde TDI bei einer Reinheit von ca. 99,1% (GC) und einer Ausbeute von 98,5% gewonnen. Der Phosgen-Hold-Up im Reaktor betrug auf Basis des eintretenden Phosgen-Massenstromes maximal ca. 38 g. Dieser Wert wurde mit der Annahme errechnet, dass das Phosgen in der Reaktion nicht verbraucht wurde. Damit ergab sich ein Phosgen-Hold-Up von ca. 76 g für eine Produktionsleistung von 1 kg TDI pro Stunde.

### Vergleichsbeispiel 3 unter Verwendung einer MCB-Phosgen-Lösung

Analog zu Beispiel 1 wurden 90 g eines Toluylendiamingemisches (TDA-Gemisch), das zu 80 Gewichts-% aus 2,4-TDA und zu 20 Gewichts-% aus 2,6-TDA bestand, in 360 g Monochlorbenzol gelöst. Die erhaltene TDA-Lösung wurde in der in Figur 2 dargestellte Mischvorrichtung auf der Achse mit einer Geschwindigkeit von 30 m/s und einem Durchsatz von 1,8 l/h bei einer Temperatur von 50°C eingedüst. Gleichzeitig wurden 1,6 kg einer 25 %-Phosgenlösung, die aus 400 g Phosgen und 1,2 kg MCB hergestellt wurde, mit einer Geschwindigkeit von 30 m/s und unter einem Winkel von 45° gegenüber der Achse über den ersten Ringspalt der Düsenmischvorrichtung bei einer Temperatur von 30°C eingedüst. Das Gemisch gelangte unmittelbar aus der Düsenmischvorrichtung in einen Rohrreaktor, der ein Volumen von ca. 375 ml und eine Länge von 30 m hatte und für das Gesamtgemisch eine Verweilzeit von ca. 2,7 min besaß und auf eine Temperatur von ca. 130° bis 140° temperiert war. Vor und nach der Synthese wurde die Apparatur kontinuierlich mit Monochlorbenzol als inertes Lösungsmittel vorgespült. Nach der destillativen Entfernung des Phosgens und des Chlorbenzols wurde TDI bei einer Reinheit von ca. 99,1 % (GC) und einer Ausbeute von 97,4% gewonnen. Der Phosgen-Hold-Up im Reaktor betrug auf Basis des eintretenden Phosgen-Massenstromes maximal ca. 72 g. Dieser Wert wurde mit der Annahme errechnet, dass das Phosgen in der Reaktion nicht verbraucht wurde. Damit ergab sich ein Phosgen-Hold-Up von ca. 147 g für eine Produktionsleistung von 1 kg TDI pro Stunde.

### Zusammenfassung der Beispiele

In nachstehender Tabelle 1 sind die Ergebnisse des Beispiels 1, sowie der Vergleichsbeispiele 2 und 3 gegenübergestellt. Aus Tabelle 1 geht deutlich hervor, dass das erfindungsgemäße Verfahren zu einer Erhöhung der Selektivität ohne Erhöhung des Phosgen-Hold-Ups oder ohne Senkung der Raum-Zeit-Ausbeute führt.

| Beispiel | Beispiel 1 | Vergleichsbeispiel 2 | Vergleichsbeispiel 3 |
|---|---|---|---|
| Phosgen-Hold-Up für 1 kg/h Produktionsleistung | 76 g | 76 g | 147 g |
| Ausbeute | 99,2 % | 98,5 % | 97,4 |
| Destillative Reinheit | 99,1 % | 99,1 % | 99,1 % |

## Patentansprüche

1. Verfahren zur Herstellung von Isocyanaten durch Umsetzung von primären Aminen mit Phosgen, wobei Isocyanat als Lösungsmittel verwendet wird, **dadurch gekennzeichnet, dass** ein Anteil oder die gesamte Menge des als Lösungsmittel verwendeten Isocyanats den Reaktionspartnern erst nach dem Zusammenführen von Amin und Phosgen zugegeben wird.

2. Verfahren nach Anspruch 1, **dadurch gekennzeichnet, dass** es in kontinuierlicher Arbeitsweise durchgeführt wird.

3. Verfahren nach Anspruch 1, **dadurch gekennzeichnet, dass** das Teile des hergestellten Isocyanats rückgeführt und als Lösungsmittel verwendet werden.

4. Verfahren nach einem der Ansprüche 1 bis 3, **dadurch gekennzeichnet, dass** der Anteil an Isocyanat, welcher der Umsetzung erst nach dem Zusammenführen von Amin und Phosgen zugegeben wird, mindestens 25 Gew.%, bezogen auf die Gesamtmenge des als Lösungsmittel verwendeten Isocyanats, beträgt.

5. Verfahren nach einem der Ansprüche 1 bis 4, **dadurch gekennzeichnet, dass** als Amin Methylendi(phenylamin) oder Toluylendiamin verwendet wird.

6. Verfahren nach einem der Ansprüche 1 bis 5, **dadurch gekennzeichnet, dass** die Umsetzung von Amin mit Phosgen in einem Rohrreaktor mit vorgeschalteter Mischdüsenvorrichtung erfolgt.

7. Verfahren nach Anspruch 6, **dadurch gekennzeichnet, dass** als Mischdüseneinrichtung eine axialsymmetrische Mischrohreinrichtung mit einer axialen Aminzufuhr und einer Phosgen- und Isocyanatzufuhr, die über zwei nicht axial angeordnete Ringspalte erfolgt, verwendet wird.

## Claims

1. A process for the production of isocyanates by reaction of primary amines with phosgene, in which isocyanate is used as solvent, wherein some or all of the isocyanate used as solvent is added to the reactants only after the amine and phosgene have been physically combined.

2. The process according to claim 1, which is carried out continuously.

3. The process according to claim 1, wherein portions of the isocyanate produced are recycled and used as solvent.

4. The process according to any of claims 1 to 3, wherein the amount of isocyanate added to the reaction only after the amine and phosgene have been physically combined is at least 25 wt %, based on the total amount of isocyanate used as solvent.

5. The process according to any of claims 1 to 4, wherein the amine used is methylenedi(phenylamine) or tolylenediamine.

6. The process according to any of claims 1 to 5, wherein the reaction of amine with phosgene is carried out in a tubular reactor having an upstream injector mixing device.

7. The process according to claim 6, wherein the injector mixing device used is an axially symmetrical mixing tube device having an axial amine feed and phosgene and isocyanate feeds effected through two off-axis annular gaps.

## Revendications

1. Procédé de préparation d'isocyanates par réaction d'amines primaires avec du phosgène, de l'isocyanate étant utilisé en tant que solvant, **caractérisé en ce qu'**une part ou la quantité totale de l'isocyanate utilisé en tant que solvant n'est ajoutée aux partenaires de réaction qu'après réunion de l'amine et du phosgène.

2. Procédé selon la revendication 1, **caractérisé en ce qu'**il est mis en oeuvre selon un mode de travail en continu.

3. Procédé selon la revendication 1, **caractérisé en ce que** les parties de l'isocyanate préparé sont recyclées et utilisées en tant que solvant.

4. Procédé selon l'une quelconque des revendications 1 à 3, **caractérisé en ce que** la part d'isocyanate qui n'est ajoutée à la réaction qu'après réunion de l'amine et du phosgène est d'au moins 25 % en poids, par rapport à la quantité totale de l'isocyanate utilisé en tant que solvant.

5. Procédé selon l'une quelconque des revendications 1 à 4, **caractérisé en ce que** de la méthylènedi(phénylamine) ou de la toluylènediamine est utilisée en tant qu'amine.

6. Procédé selon l'une quelconque des revendications 1 à 5, **caractérisé en ce que** la réaction de l'amine avec le phosgène a lieu dans un réacteur tubulaire avec un dispositif de buse mélangeur placé en amont.

7. Procédé selon la revendication 6, **caractérisé en ce qu'**un équipement de tube mélangeur à symétrie axiale avec un apport axial d'amine et un apport de phosgène et d'isocyanate ayant lieu par l'intermédiaire de deux fentes annulaires disposées de manière non axiale est utilisé en tant que dispositif de buse mélangeur.
